# EUROPEAN PATENT SPECIFICATION

(11) **EP 4 230 997 B1**
(45) Date of publication and mention of the grant of the patent: **23.09.2026**
(21) Application number: 21880023.3
(22) Date of filing: 08.10.2021
(51) Int. Cl.: G01N 21/31, G01N 21/47, G01N 33/02, G01N 21/33, G01N 21/359, G01N 21/84

(54) **MOBILE APPARATUS, FOOD CHARACTERISTICS DETERMINATION DEVICE, FOOD CHARACTERISTICS DETERMINATION METHOD, AND PROGRAM**
MOBILE VORRICHTUNG, VORRICHTUNG ZUR BESTIMMUNG VON LEBENSMITTELEIGENSCHAFTEN, VERFAHREN ZUR BESTIMMUNG VON LEBENSMITTELEIGENSCHAFTEN UND PROGRAMM
APPAREIL MOBILE, DISPOSITIF ET PROCÉDÉ DE DÉTERMINATION DES CARACTÉRISTIQUES DES ALIMENTS, ET PROGRAMME

(30) Priority: 13.10.2020 JP 2020172779
(43) Date of publication of application: 23.08.2023
(73) Proprietor: RIKEN, Wako-shi, Saitama 351-0198 (JP)
(72) Inventor: YUMOTO, Masaki, Wako-shi, Saitama 351-0198 (JP); WADA, Satoshi, Wako-shi, Saitama 351-0198 (JP); OGAWA, Takayo, Wako-shi, Saitama 351-0198 (JP); MURAKAMI, Takeharu, Wako-shi, Saitama 351-0198 (JP); SAKASHITA, Michio, Wako-shi, Saitama 351-0198 (JP); SHINJO, Atsushi, Wako-shi, Saitama 351-0198 (JP)
(74) Representative: Patentanwälte Olbricht Buchhold Keulertz
(86) International application number: PCT/JP2021/037472
(87) International publication number: WO 2022/080284

(56) References cited:
- WO-A1-2018/038052
- CN-A- 111 665 201
- JP-A- 2011 048 591
- JP-A- 2012 103 092
- JP-A- 2018 096 834
- JP-A- 2020 101 409
- KR-A- 20190 114 775
- US-A1- 2016 150 213
- US-A1- 2018 172 510
- US-A1- 2018 172 510
- US-B2- 10 458 845

## Description

### BACKGROUND

### 1. TECHNICAL FIELD

The present invention relates to a mobile apparatus, a food characteristics determination device, a food characteristics determination method, and a program.

### 2. RELATED ART

In Patent document 1, it is described as "the mobile terminals (301 to 303) are equipped with an optical sensor that can measure the sugar content and maturity of fruit products, and when the owner of the mobile terminal holds the mobile phone over a fruit product at the storefront, at the same time as the kind of the fruit product is recognized, measurement of sugar content and maturity is started, and the owner of the mobile terminal can be notified of the degree of deliciousness corresponding to the kind of the fruit product based on the measured sugar content and maturity."
Further US 2018/0172510 A1, US 10,458,845 B2 and US 2016/0150213 A1 relate to systems and methods for identification or spectrometry, especially for food. Prior Art Document

### Patent document

Patent document 1: Japanese Patent Application Publication No. 2011-48591

### SUMMARY

In the first aspect of the present invention, provided is a mobile apparatus according to appended claim 1.

The mobile apparatus may be a general-purpose information communication terminal configured to fulfill a function corresponding to an installed application. The spectroscope may be detachable. The radiating unit may be detachable.

An image-capturing unit configured to generate an image by image-capturing may further be comprised. The identification unit may be configured to identify the kind of the food product based on the image.

The storage unit may be configured to store the information about light in further association with a part of the food product. The identification unit may be configured to identify the part of the food product based on the image. The determination unit may be configured to determine the characteristic of the food product further based on the part of the food product.

The characteristic of the food product may include at least any of color, sugar content, acidity, maturity, firmness, or a functional ingredient amount. The radiating unit may be configured to radiate light in the visible light band, and the spectroscope may be configured to have sensitivity to the visible light band. The radiating unit may be configured to radiate light in the near-infrared band, and the spectroscope is configured to have sensitivity to the near-infrared band. The radiating unit may be configured to radiate light in the ultraviolet band, and the spectroscope is configured to have sensitivity to the ultraviolet band.

The storage unit may be configured to store the information about light in further association with the distinction between scattered light and transmitted light. The determination unit may be configured to determine the characteristic of the food product further based on whether the measured value is of scattered light or transmitted light.

In the second aspect of the present invention, provided is a food characteristics determination device, comprising: a storage unit configured to store a kind of food product, a characteristic of the food product, and information about light in association with each other; a communication unit configured to communicate with a mobile apparatus; and a determination unit configured to determine a characteristic of a food product by acquiring a kind of the food product and a measured value of spectroscopy from the mobile apparatus via the communication unit and referring to the storage unit, and output the characteristic of the food product to the mobile apparatus via the communication unit.

In the third aspect of the present invention, provided is a food characteristics determination method, comprising: radiating light; performing spectroscopy of light entered to acquire a measured value; identifying a kind of a food product corresponding to the light entered in the performing the spectroscopy; and determining a characteristic of the food product to output the characteristic of the food product, by referring to a storage unit configured to store a kind of a food product, a characteristic of the food product, and information about light in association with each other, based on the measured value output in the performing the spectroscopy and the kind of the food product identified in the identifying, to determine the characteristic of the food product.

In the fourth aspect of the present invention, provided is a program for causing a mobile apparatus to perform: radiating light from a radiating unit; performing spectroscopy of light entered to acquire a measured value from a spectroscope; identifying a kind of a food product corresponding to the light entered in the performing the spectroscopy; and determining a characteristic of the food product to output the characteristic of the food product, by referring to a storage unit configured to store a kind of a food product, a characteristic of the food product, and information about light in association with each other, based on the measured value output in the performing the spectroscopy and the kind of the food product identified in the identifying, to determine the characteristic of the food product.

The summary clause does not necessarily describe all necessary features of the embodiments of the present invention. The invention may also include a subcombination of the features described above.

### BRIEF DESCRIPTION OF THE DRAWINGS

Fig. 1 schematically shows the exterior of a mobile apparatus 10.
Fig. 2 schematically shows the exterior of a mobile apparatus 10.
Fig. 3 schematically shows function blocks of a mobile apparatus 10.
Fig. 4 shows an example of information stored in a storage unit 150.
Fig. 5 shows an example of operation flow S10 of the mobile apparatus 10.
Fig. 6 schematically shows the exterior of another mobile apparatus 12.
Fig. 7 schematically shows function blocks of still another mobile apparatus 16.
Fig. 8(a) schematically shows the exterior of the front of still another mobile apparatus 50.
Fig. 8(b) schematically shows the exterior of a side of still another mobile apparatus 50.
Fig. 9(a) schematically shows the exterior of the front of still another mobile apparatus 52.
Fig. 9(b) schematically shows the exterior of a side of still another mobile apparatus 52.
Fig. 10(a) schematically shows the exterior of the front of a mobile apparatus 54 in accordance with the claimed invention.
Fig. 10(b) schematically shows the exterior of a side of the mobile apparatus 54.
Fig. 11(a) schematically shows the exterior of the front of another mobile apparatus 56 in accordance with the claimed invention.
Fig. 11(b) schematically shows the exterior of a side of the mobile apparatus 56.
Fig. 12 schematically shows function blocks of a food characteristics determination system 22 according to the second embodiment.
Fig. 13(a) shows an example of determining "Color 1" as a characteristic of "Tomato" as a kind of a food product using the mobile apparatus 10 according to the first embodiment.
Fig. 13(b) shows an example of determining "Color 1" as a characteristic of "Tomato" as a kind of a food product using the mobile apparatus 10 according to the first embodiment.
Fig. 13(c) shows an example of determining "Color 1" as a characteristic of "Tomato" as a kind of a food product using the mobile apparatus 10 according to the first embodiment.
Fig. 14 shows an example of determining "Sugar content" as a characteristic of "Muscat" as a kind of a food product using the mobile apparatus 10 according to the first embodiment.
Fig. 15 shows an example of determining "Acidity" as a characteristic of "Muscat" as a kind of a food product using the mobile apparatus 10 according to the first embodiment.

### DESCRIPTION OF EXEMPLARY EMBODIMENTS

Hereinafter, the present invention will be described through embodiments of the present invention, but the following embodiments do not limit the present invention according to claims. In addition, not all combinations of features described in the embodiment are essential to the solution of the invention.

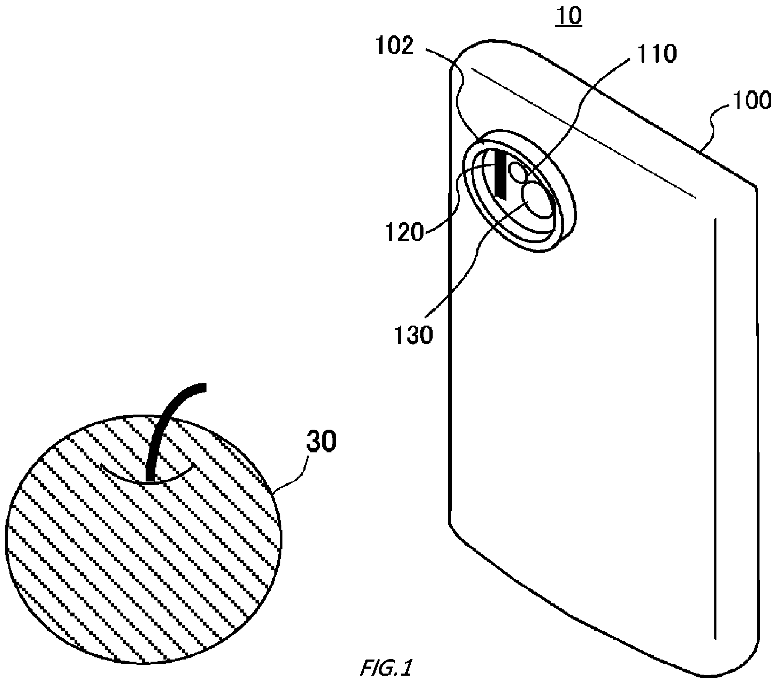

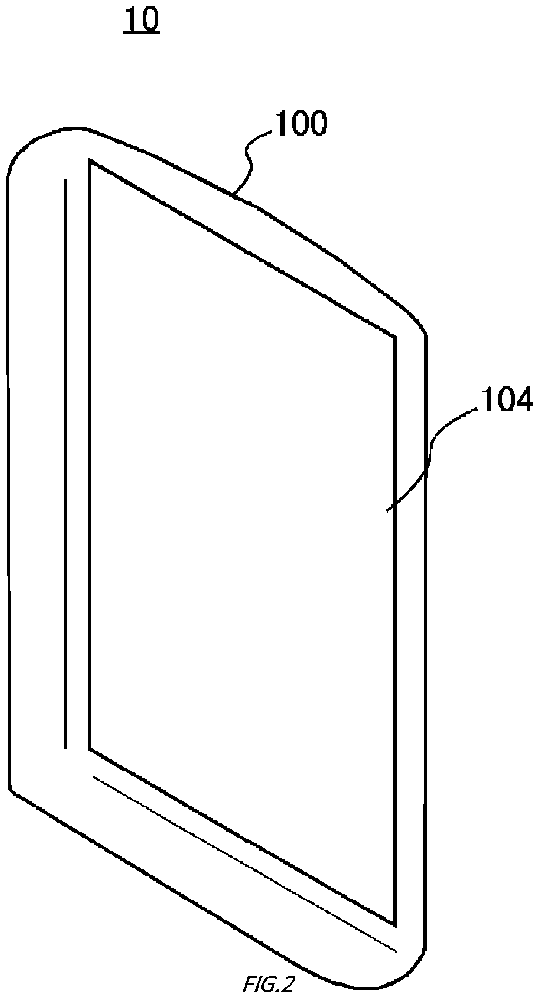

Fig. 1 and Fig. 2 schematically show the exterior of a mobile apparatus 10. The mobile apparatus 10 is configured to determine a characteristic, such as sugar content, of a food product 30, such as a greengrocery product.

The mobile apparatus 10 is a general-purpose information communication terminal, such as a smartphone, a tablet, and a PDA, configured to fulfill the function corresponding to the installed application. On one surface side of a body 100 of the mobile apparatus 10, a touch panel 104 configured to display information and receive input from the user is provided.

On the other surface side of the mobile apparatus 10, a sample holder 102 having a cylindrical shape is provided. In the sample holder 102, a radiating unit 110, a spectroscope 120, and an image-capturing unit 130 are provided. The inner diameter of the cylindrical shape is approximately 3 cm to 4 cm, and the height is approximately 2 cm to 3 cm. In the sample holder 102, an optical system such as a lens may further be arranged.

The radiating unit 110 includes a source of light such as an LED and is configured to radiate light in the visible light band, the near-infrared band, and the ultraviolet band. Light in each band may be radiated at the same time.

The spectroscope 120 is configured to perform spectroscopy of light entered to output a measured value. For example, the spectroscope 120 is configured to perform spatial wavelength dispersion of light entered from a slit using a reflective grating and receive the light using a photodetector, to output the intensity of the light, which is associated with a wavelength, as a measured value. The spectroscope is small as an approximately 1 cm cube, and a mini-spectrometer of "Micro series" or "SMD series" made by Hamamatsu Photonics K.K. or the like may be used, for example.

The image-capturing unit 130 is what is called a digital camera module having an optical system and an image-capturing device. The image-capturing unit 130 is configured to generate an image by image-capturing. For example, an image of the food product 30 is generated by capturing an image of the food product 30.

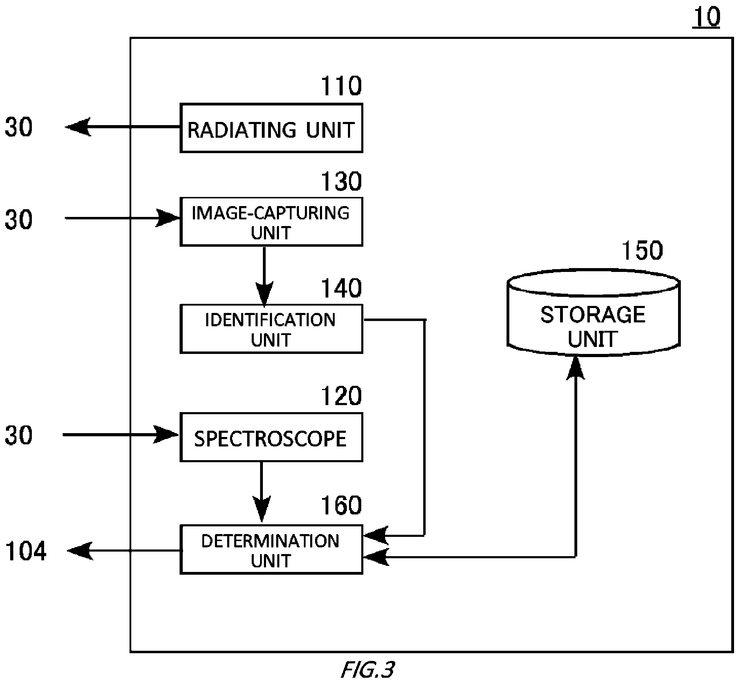

Fig. 3 schematically shows function blocks of the mobile apparatus 10. In addition to the radiating unit 110, the spectroscope 120, and the image-capturing unit 130 described above, the mobile apparatus 10 comprises an identification unit 140, a storage unit 150, and a determination unit 160.

The identification unit 140 is configured to identify the kind of the food product 30 corresponding to the light entered the spectroscope 120. In this case, the identification unit 140 is configured to identify the kind of the food product 30 based on the image generated by the image-capturing unit 130. The identification unit 140 may identify the kind of the food product based on the matching degree with each of the pre-stored feature quantities of the food product, by image analysis, or may identify the kind of the food product using machine learning such as supervised learning Convolutional Neural Network (CNN) where pictures of food products and the kinds are used for the learning in advance.

The storage unit 150 is configured to store a kind of food product, a characteristic of the food product, and information about light in association with each other. The determination unit 160 is configured to determine a characteristic of the food product to output the characteristic of the food product, by referring to the storage unit 150, based on the measured value output from the spectroscope 120 and the kind of the food product identified by the identification unit 140.

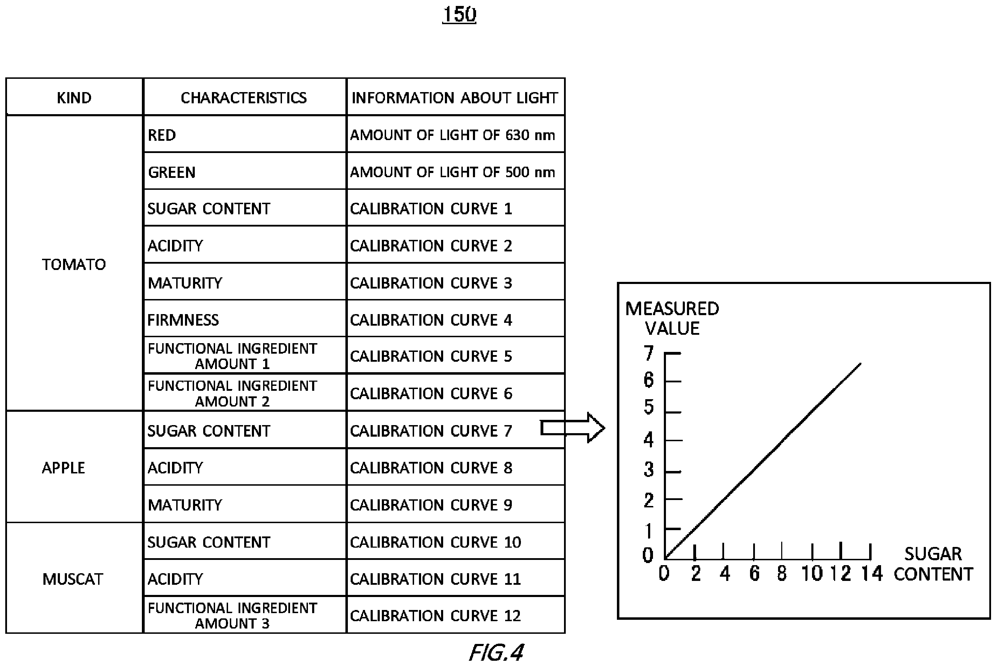

Fig. 4 shows an example of information stored in the storage unit 150. As described above, the kinds of food products, characteristics of the food products, and information about light are associated with each other.

The kinds of food products are greengrocery products such as a tomato, an apple, and a pumpkin, for example. The characteristics of food products include at least any of color, sugar content, acidity, maturity, firmness, or a functional ingredient amount. In the example in Fig. 4, in association with "Tomato" as a kind of a food product, color 1, color 2, sugar content, acidity, maturity, firmness, functional ingredient amount 1, and functional ingredient amount 2 are associated as characteristics of the food product. Examples of color 1 and 2 are red and green. Examples of functional ingredient amount 1 and 2 are the amount of chlorophyll and the amount of lycopene. The characteristic of the food product to be determined may be different for each kind of the food product. In the example in Fig. 4, although sugar content, acidity, and maturity are associated with "Apple" as a kind of a food product, functional ingredient amount 1 and 2 are not associated with it.

The information about light may be an amount of light at a specific wavelength such as for color 1 or may be a calibration curve representing the correlation between a value of a food product and a measured value such as for the sugar content. In the example in Fig. 4, information about light for color 1 is an amount of light of 630 nm, which corresponds to the red range. The information about light for color 2 is an amount of light of 500 nm, which corresponds to the green range.

Fig. 4 schematically shows, as an example of the calibration curve, calibration curve 7 for the sugar content of "Apple" as a kind of a food product. Here, the horizontal axis shows the magnitude of sugar content as a value of a characteristic. The vertical axis shows the magnitude of a measured value. This measured value is obtained by converting a measured value output from the spectroscope 120 by using a predetermined calculation formula. The conversion formula and/or calibration curve of a measured value is/are calculated in advance experimentally, through simulation and/or machine learning, such as multivariate analysis and principal component analysis.

Additionally, the magnitude of the measured value depends on the radiation characteristics of the radiating unit 110 and the sensitivity characteristics of the spectroscope 120. Thus, the calibration curve described above can also be described as what is obtained by performing calibration on a set of a specific type of radiating unit 110 and a specific type of spectroscope 120. Therefore, a calibration curve as a standard may temporarily be stored in the storage unit 150, and the user may be prompted to use the radiating unit 110 to radiate light and use the spectroscope 120 to perform measurement for a standard sample, and based on the measured value, calibration may be performed on the calibration curve for this mobile apparatus.

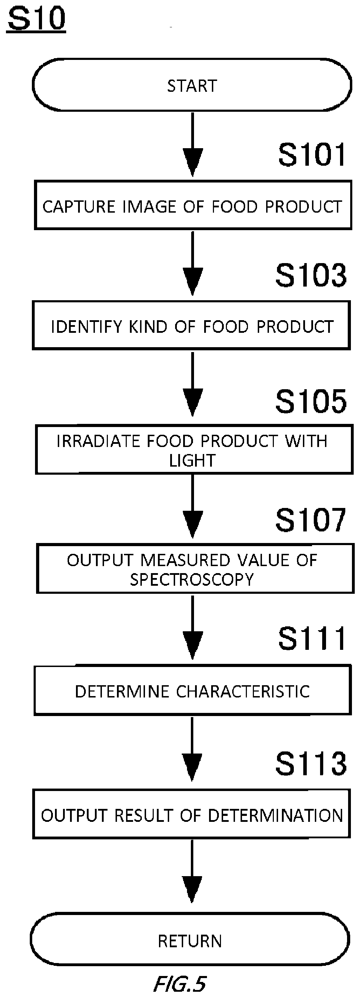

Fig. 5 shows an example of operation flow S10 of the mobile apparatus 10. Operation flow S10 starts on the mobile apparatus 10 when an application configured to determine a characteristic of a food product is activated by the user. Said application is a computer program configured to cause the mobile apparatus 10 to perform each step of operation flow S10.

The mobile apparatus 10 displays a message to prompt the user to capture an image of the food product 30 on the touch panel 104 (S101). In this case, a frame together with the message "fit the entire food product within the frame" may be displayed on the touch panel to facilitate identifying the kind of the food product 30. When the user presses down a shooting button, the image-capturing unit 130 captures an image of the food product 30 and generates an image of the food product 30 (the same step). The identification unit 140 identifies the kind of the food product 30 based on the image generated by the image-capturing unit 130 (S103).

The radiating unit 110 irradiates the food product 30 with light (S105). In this case, to avoid the impact of external light, a message to prompt the user to cause the food product 30 to contact with the sample holder 102 is preferably displayed. In the present example, the radiating unit 110 radiates light in all bands of near-infrared, visible light, and ultraviolet that the radiating unit 110 itself can emit, regardless of the kind of the food product 30, the corresponding characteristic, and information about light.

The spectroscope 120 performs spectroscopy of the light entered to output a measured value (S107). In this case, during the radiation of light by the radiating unit 110, the spectroscope 120 outputs the measured value. In this way, scattered light which is light radiated by the radiating unit 110 and scattered by the food product 30 can be measured. Further, in the present example, the spectroscope 120 outputs a spectroscopic profile representing the intensity of each wavelength of the light entered as the measured value, regardless of the kind of the food product 30, the corresponding characteristic, and information about light.

The determination unit 160 determines a characteristic of the food product 30, by referring to the storage unit 150, based on the measured value output from the spectroscope 120 and the kind of the food product 30 identified by the identification unit 140 (S111). In the present example, the determination unit 160 also performs determination on any of the characteristics in association with the kind of the food product 30 in the storage unit 150. For example, in the example in Fig. 4, determination is sequentially performed on the sugar content, acidity, and maturity for the kind of the food product 30 "Apple".

In this case, determination unit 160 refers to the storage unit 150 and performs conversion into a measured value corresponding to the information about light for each characteristic from the profile being the measured value output from the spectroscope 120, as needed. For example, for "Sugar content" as a characteristic of the kind of the food product 30 "Apple", the formula described above with reference to Fig. 4 is used to perform the conversion. For the measured value after the conversion, the determination unit 160 determines the characteristic represented by the calibration curve. In the example in Fig. 4, when the measured value after the conversion for "Sugar content" as a characteristic of the kind of the food product 30 "Apple" is "3", sugar content is determined as "6".

The determination unit 160 outputs the result of determination to the touch panel 104 (S113). For example, the determination unit 160 displays the message indicating "measured sugar content of the apple is 6" on the touch panel 104. Operation flow S10 ends here.

Thus, according to the present example, the mobile apparatus 10, such as a smartphone, enables the user to know information about a characteristic of the food product 30 easily. In particular, using the radiating unit 110 and spectroscope 120 with a broad wavelength band enables knowing various characteristics of the food product 30.

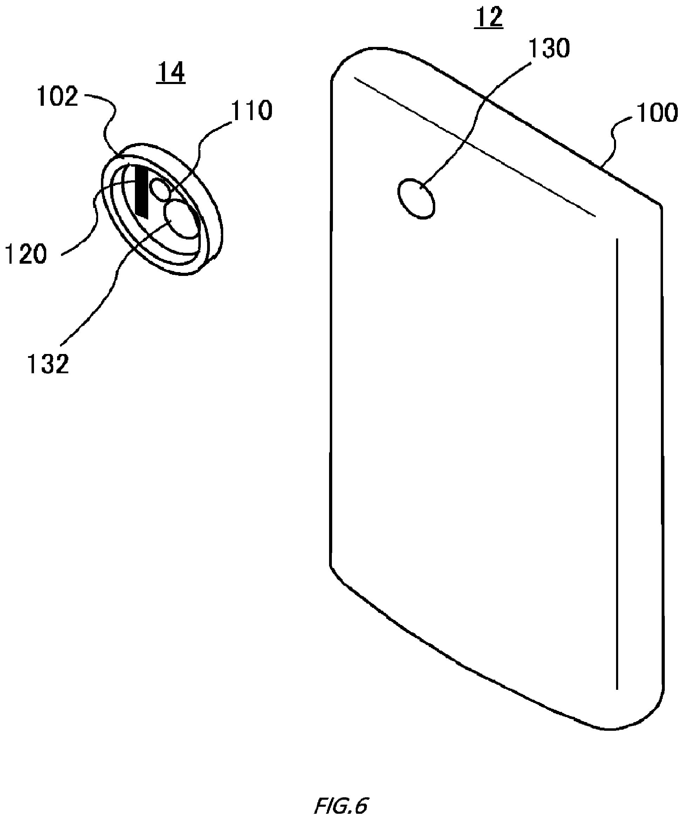

Fig. 6 schematically shows the exterior of another mobile apparatus 12. The mobile apparatus 12 is the same as the mobile apparatus 10 except for parts that will be described in particular.

The mobile apparatus 12 includes a spectroscopy unit 14 detachable from the body 100. The mobile apparatus 12 is configured to operate as a general-purpose information communication terminal even when the spectroscopy unit 14 is not attached thereto.

The spectroscopy unit 14 has the sample holder 102, the radiating unit 110, and the spectroscope 120. For the spectroscopy unit 14, a through hole 132 is further provided. The through hole 132 is arranged at a position where the image-capturing unit 130 of the body 100 looks into the through hole 132 when the spectroscopy unit 14 is attached to the body 100.

The spectroscopy unit 14 is fixed to the body 100 by means of a magnet, screwing, a clip, or the like. The spectroscopy unit 14 and the body 100 are configured to transmit and receive information through wireless communication such as Bluetooth (registered trademark). Instead of wireless communication, wired communication may be employed for transmitting and receiving information, where a connector provided for the spectroscopy unit 14 is inserted into an interface for a wire, such as a USB-c type, provided for the body 100.

Thus, when the spectroscopy unit 14 is not used, it can be detached, which allows the mobile apparatus 12 to have its effect contributing to the miniaturization of the mobile apparatus 12, in addition to the effects of the mobile apparatus 10. Note that, instead of the entire spectroscopy unit 14 being detachable, the radiating unit 110 and/or the spectroscope 120 may be detachable from the body 100 individually. In addition, a part of the radiating unit 110, a source of light in the near-infrared band and/or the ultraviolet band may be detachable.

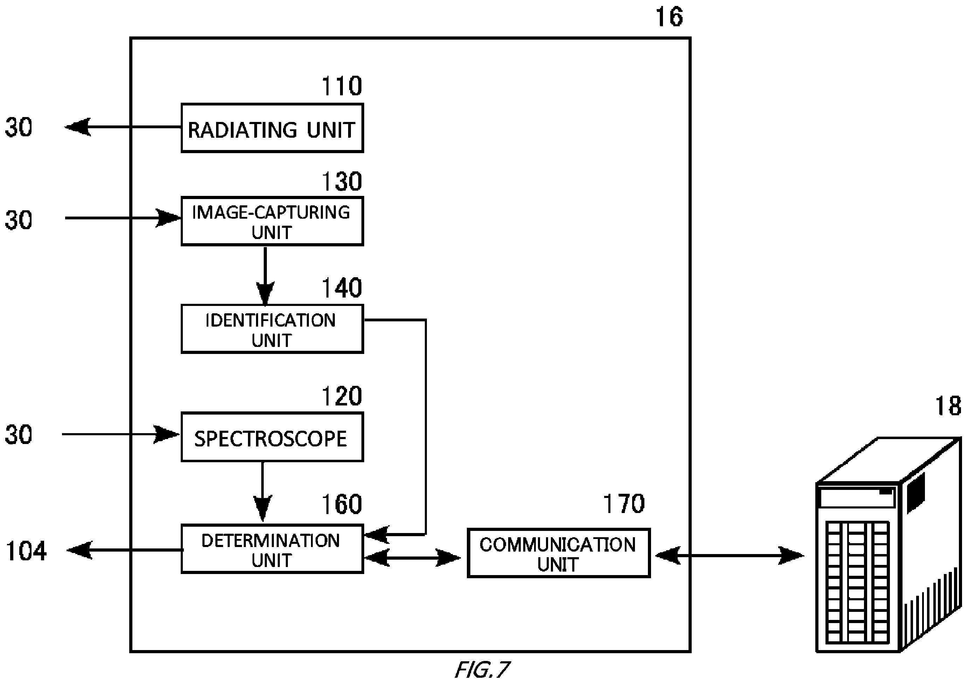

Fig. 7 schematically shows function blocks of still another mobile apparatus 16. The mobile apparatus 16 is the same as the mobile apparatus 10 except for parts that will be described in particular.

In the case of the mobile apparatus 16, the information stored in the storage unit 150 of the mobile apparatus 10 is stored in an external server 18. The mobile apparatus 16 is configured to refer to the information stored in the server 18 by communicating with the server 18 via a communication unit 170. The server 18 is a server serving the Agricultural Data Collaboration Platform (WAGRI), for example.

Thus, the mobile apparatus 16 has its effect of allowing utilizing the latest, large amounts of information stored in the server 18, in addition to the effects of the mobile apparatus 10. Note that, some pieces of the information stored in the storage unit 150 of the mobile apparatus 10, such as a piece of information that may be often used and a piece of information that was used in the past, may be stored in the mobile apparatus 16.

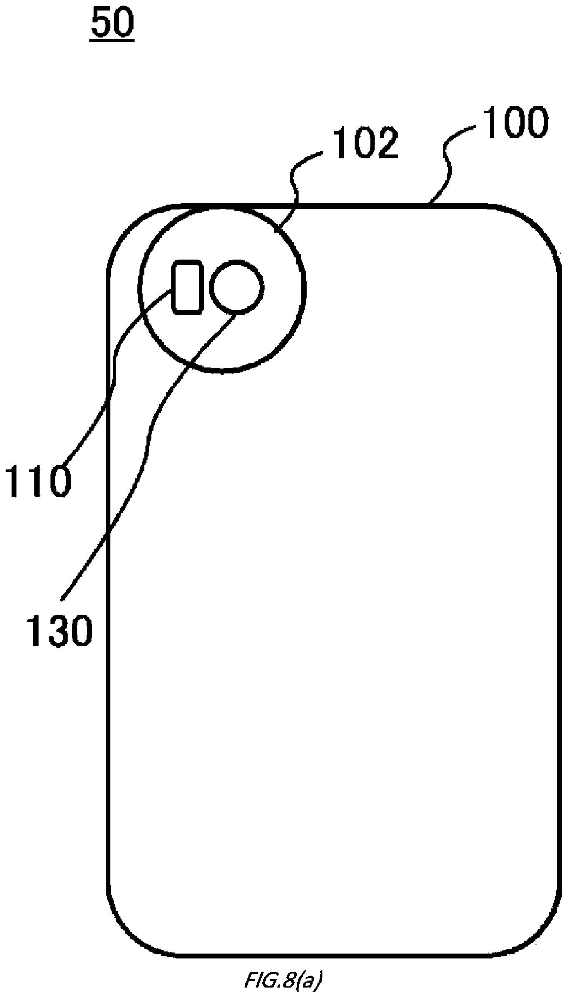

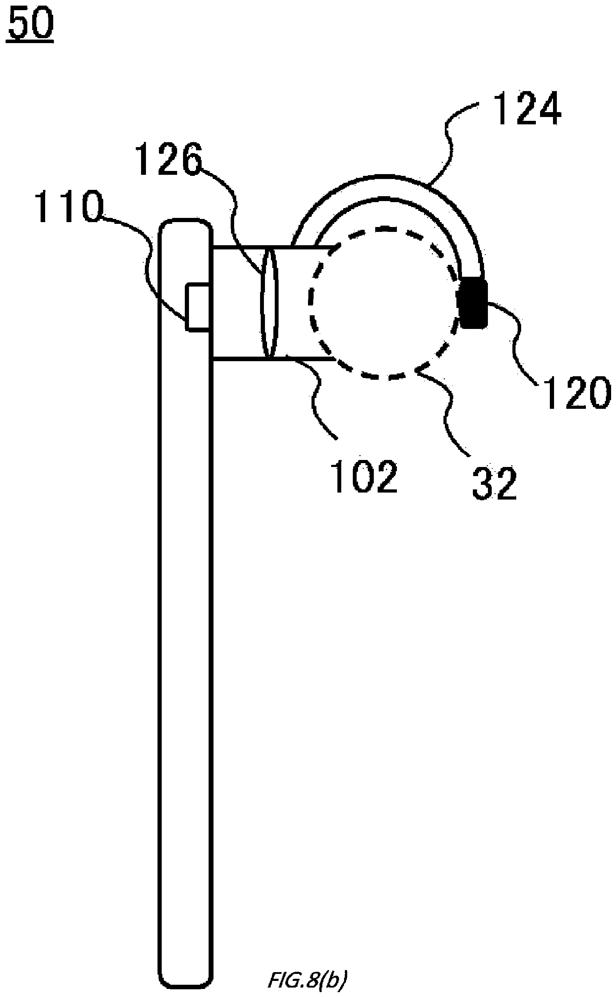

Fig. 8 (a) and Fig. 8(b) schematically show the exteriors of the front and a side of still another mobile apparatus 50. The mobile apparatus 50 is the same as the mobile apparatus 10 except for parts that will be described in particular.

In the mobile apparatus 50, the spectroscope 120 is arranged in an opposing position to the radiating unit 110 by an arm 124 having an arc shape extending from the sample holder 102. This allows the spectroscope 120 to perform spectroscopy and measurement of specularly transmitted light, which is light of the radiating unit 110 transmitted through the food product 32. In this case, information in the storage unit 150 in Fig. 4 is preferably information corresponding to measured quantities obtained by performing spectroscopy of the specularly transmitted light. Note that, the arm 124 may be hard so that the shape does not change or may be soft so that the shape can change. Note that, in the sample holder 102, an optical system 126 such as a lens is arranged.

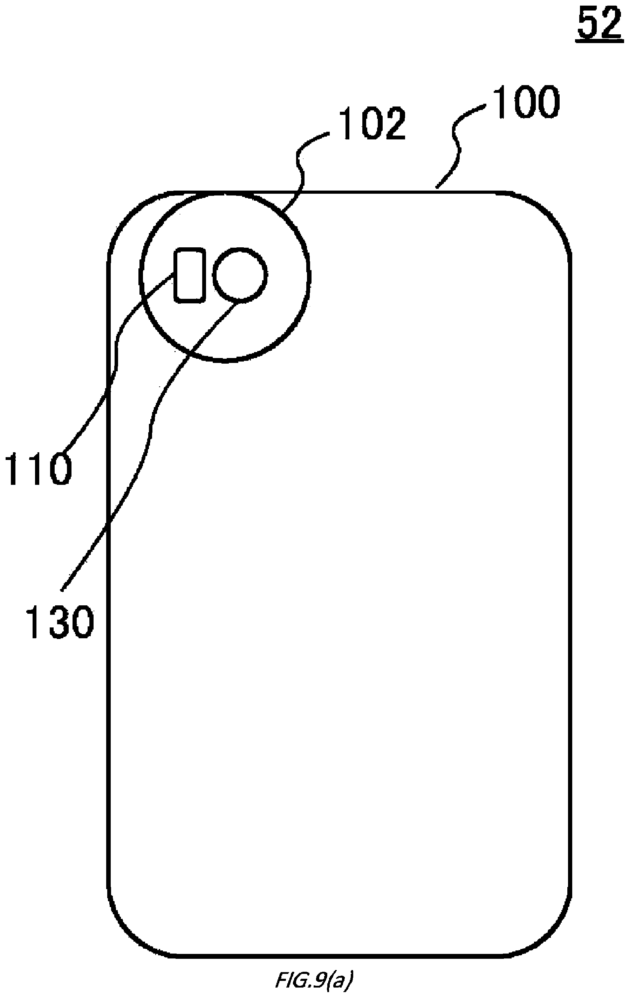

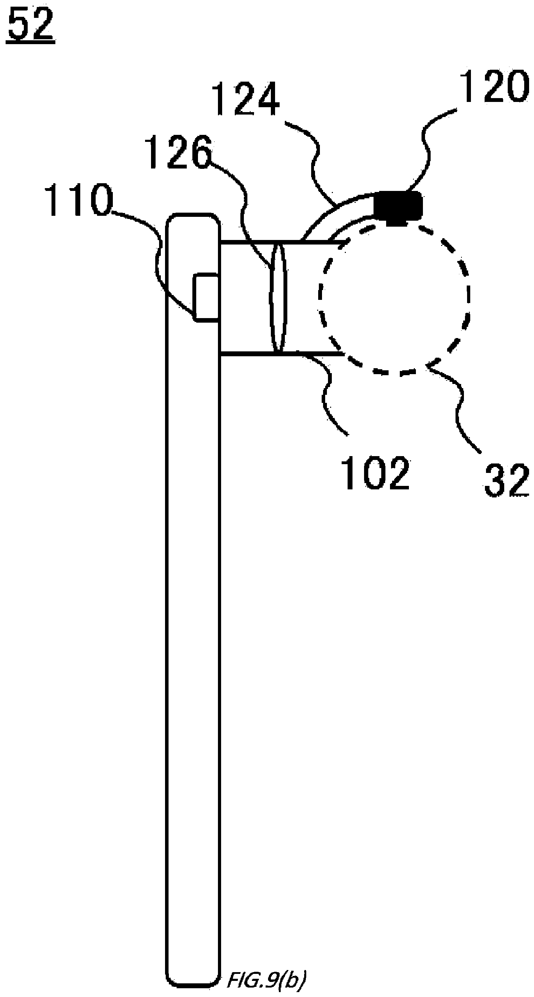

Fig. 9(a) and Fig. 9(b) schematically show the exteriors of the front and a side of still another mobile apparatus 52. The mobile apparatus 52 is the same as the mobile apparatus 50 except for parts that will be described in particular.

In the mobile apparatus 52, the spectroscope 120 is arranged sideways relative to the radiating unit 110 by the arm 124 having an arc shape extending from the sample holder 102. This allows the spectroscope 120 to perform spectroscopy and measurement of diffusely transmitted light, which is light of the radiating unit 110 transmitted through the food product 32. In this case, information of the storage unit 150 in Fig. 4 is preferably information corresponding to measured quantities obtained by performing spectroscopy of the diffusely transmitted light.

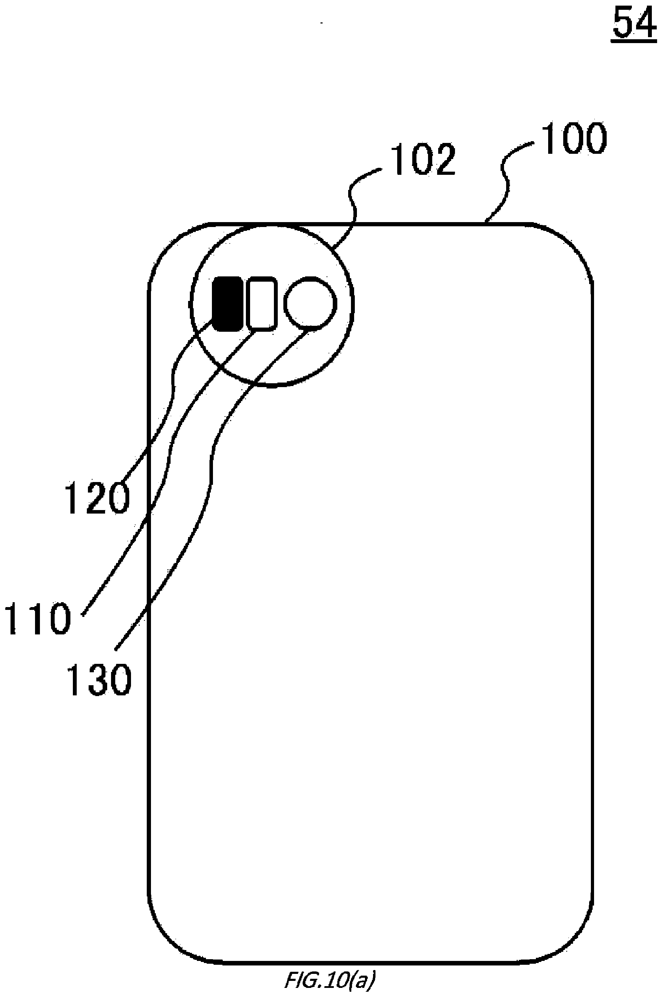

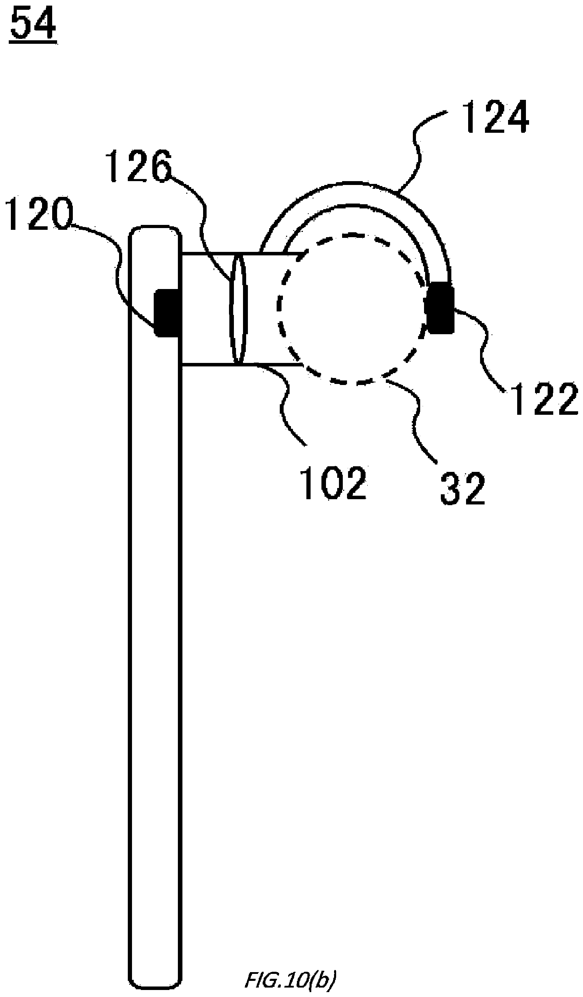

Fig. 10(a) and Fig. 10(b) schematically show the exteriors of the front and a side of a mobile apparatus 54 in accordance with the claimed invention. The mobile apparatus 54 is the same as the mobile apparatus 10 except for parts that will be described in particular.

In the mobile apparatus 54, in addition to the spectroscope 120, another spectroscope 122 is provided, and the spectroscope 122 is arranged in an opposing position to the radiating unit 110 by the arm 124 having an arc shape extending from the sample holder 102. This allows the spectroscope 120 to perform spectroscopy of scattered light of the food product 32 and additionally allows the spectroscope 122 to perform spectroscopy and measurement of specularly transmitted light transmitted through the food product 32.

In this case, information about light in the storage unit 150 in Fig. 4 is preferably stored in further association with the distinction between scattered light and specularly transmitted light. The determination unit 160 is configured to determine a characteristic of the food product 32 further based on whether a measured value is of scattered light or specularly transmitted light.

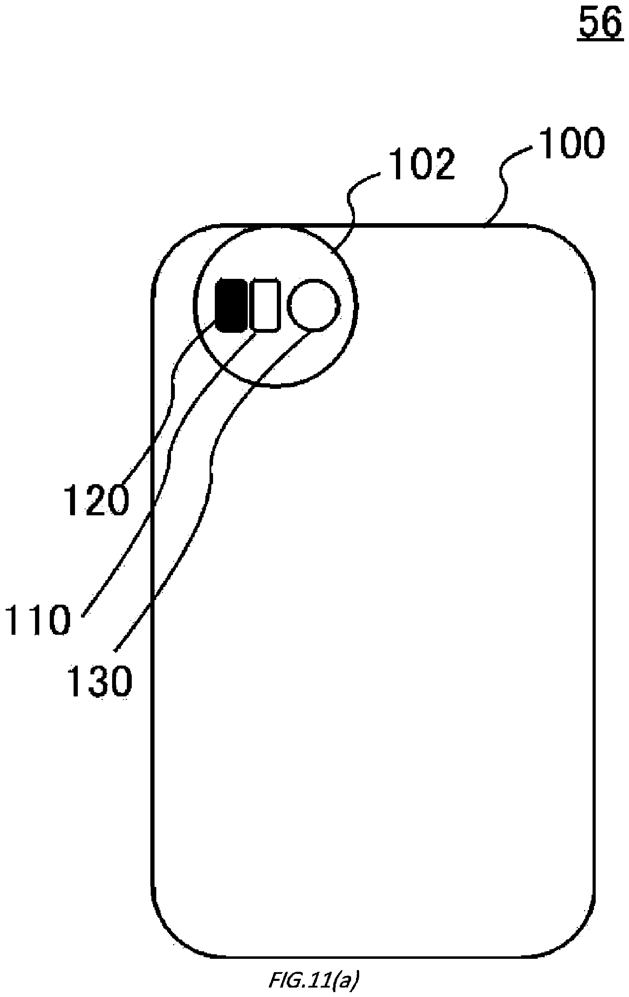

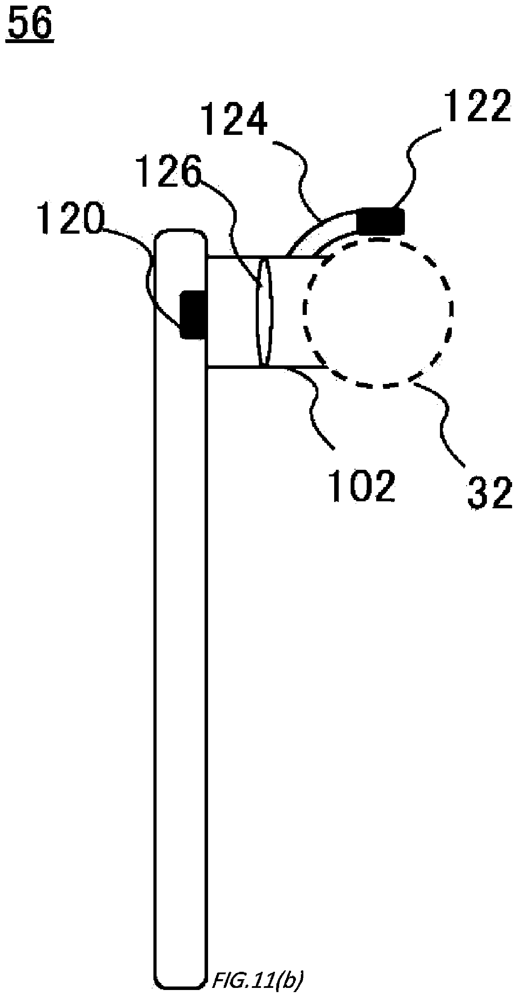

Fig. 11(a) and Fig. 11(b) schematically show the exteriors of the front and a side of another mobile apparatus 56 in accordance with the claimed invention. The mobile apparatus 56 is the same as the mobile apparatus 54 except for parts that will be described in particular.

In the mobile apparatus 56, in addition to the spectroscope 120, another spectroscope 122 is provided, and the spectroscope 122 is arranged sideways relative to the radiating unit 110 by the arm 124 having an arc shape extending from the sample holder 102. This allows the spectroscope 120 to perform spectroscopy of scattered light of the food product 32 and additionally allows the spectroscope 122 to perform spectroscopy and measurement of diffusely transmitted light transmitted through the food product 32.

In this case, information about light in the storage unit 150 in Fig. 4 is preferably stored in further association with the distinction between scattered light and diffusely transmitted light. The determination unit 160 is configured to determine a characteristic of the food product 32 based on whether a measured value is of scattered light or diffusely transmitted light.

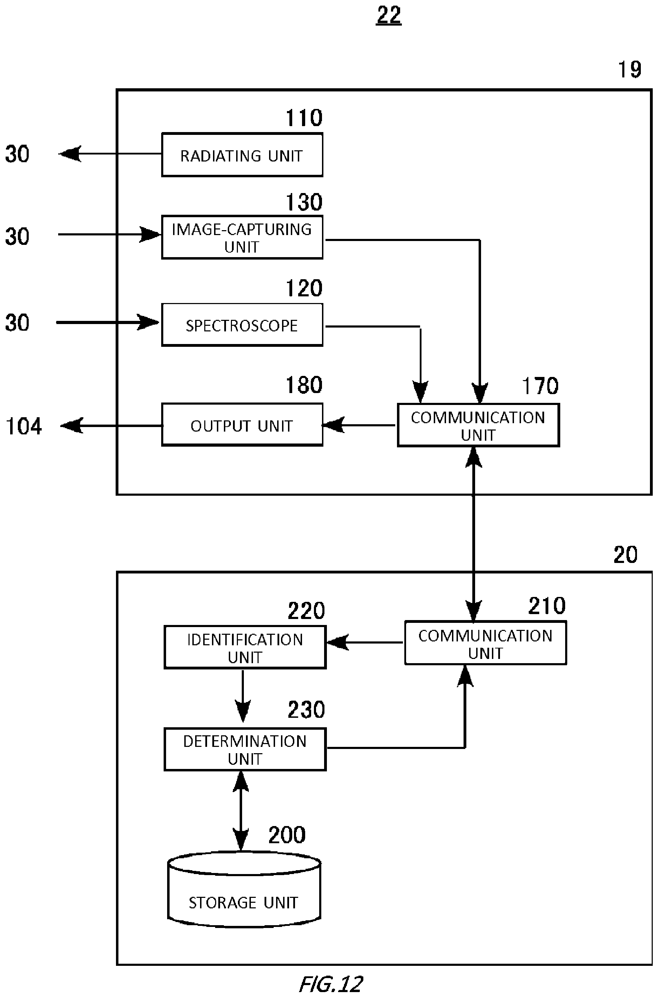

Fig. 12 schematically shows function blocks of a food characteristics determination system 22 according to the second embodiment. The food characteristics determination system 22 comprises a mobile apparatus 19 and a food characteristics determination device 20 in communication with each other through the Internet or the like. The mobile apparatus 19 is the same as the mobile apparatus 10 except for parts that will be described in particular.

In the mobile apparatus 19, the image-capturing unit 130 is configured to transmit an image of the food product 30 to the food characteristics determination device 20 via the communication unit 170. Similarly, the spectroscope 120 is configured to transmit a measured value of the spectroscopy of the food product 30 via the communication unit 170. In this case, information associating them with each other and information required to return information to the mobile apparatus 19 are also transmitted.

The food characteristics determination device 20 is, for example, a general-purpose information processing device, such as a personal computer, with a program installed therein. The food characteristics determination device 20 may also be a server serving the WAGRI described above. The food characteristics determination device 20 comprises a storage unit 200, a communication unit 210, an identification unit 220, and a determination unit 230.

The storage unit 200 has information similar to the information stored in the storage unit 150 of the mobile apparatus 10. In other words, the storage unit 200 is configured to store a kind of a food product, a characteristic of the food product, and information about light in association with each other. The communication unit 210 is configured to communicate with the mobile apparatus 19.

The identification unit 220 is configured to perform an operation similar to the operation of the identification unit 140 of the mobile apparatus 10. In other words, the identification unit 220 is configured to identify the kind of the food product 30 based on an image of the food product 30 acquired via the communication unit 210. Instead, the identification unit 140 may be provided on the side of the mobile apparatus 19, and the information indicating the kind of the food product 30 identified by the identification unit 140 may be acquired by the food characteristics determination device 20 via the communication unit 210.

The determination unit 230 is configured to perform an operation similar to the operation of the determination unit 160 of the mobile apparatus 10. In other words, the determination unit 230 is configured to determine a characteristic of a food product by referring to the storage unit 200, based on the kind of the food product 30 identified by the identification unit 220 and a measured value of spectroscopy acquired from the mobile apparatus 19. The determination unit 230 is configured to output the result of the determined characteristic to the mobile apparatus 19 via the communication unit 210. An output unit 180 of the mobile apparatus 19 is configured to display, on the touch panel 104, information on the determined characteristic of the food product 30 acquired from the food characteristics determination device 20 via the communication unit 170.

Thus, according to the second embodiment, in addition to the effects of the first embodiment, an effect of reducing the computational load in the mobile apparatus 19 can be obtained. Note that, information about light may be stored in association with information for identifying the radiating unit 110 and the spectroscope 120 in the storage unit 200, and the determination unit 230 may further receive information for identifying the radiating unit 110 and the spectroscope 120 from the mobile apparatus 19 to determine a characteristic of the food product 30 based on said information.

In any of the embodiments described above, the identification units 140, 220 may identify, based on an image of the food product 30, a part of the food product 30, for example, whether the part is closer to or far from the calyx of the greengrocery product. In this case, in the storage units 150, 200, information about light is stored in further association with a part of a food product, and the determination unit is configured to determine a characteristic of the food product further based on the part of the food product. This enables determining a characteristic of a food product further precisely.

In any of the embodiments described above, the identification units 140, 220 are configured to identify the kind of the food product 30 based on an image of the food product 30. Instead, or in addition, the user may input the kind of the food product 30. In this case, the kinds of food products stored in the storage unit 150 may be displayed in a selectable way on the touch panel 104.

In any of the embodiments described above, the determination units 160, 230 are configured to determine any of the characteristics associated with the kind of the food product 30. Instead, or in addition, the user may select the characteristic to be determined. In this case, characteristics of a food product stored in the storage unit 150 may be displayed in a selectable way on the touch panel 104. In addition, if the kind of the food product 30 has already been identified, the storage unit 150 may be referred, and characteristics corresponding to the identified kind may be displayed in a selectable way on the touch panel 104.

In any of the embodiments described above, the radiating unit 110 is configured to radiate light in the near infrared range, the visible light range, and the ultraviolet band. Accordingly, the spectroscopes 120, 122 have sensitivity to the near infrared range, the visible light range, and the ultraviolet band. Instead, only light in a band corresponding to a measured value corresponding to a characteristic to be determined may be radiated. Also, only a measured value corresponding to a characteristic to be determined may be output from the spectroscopes 120, 122.

Also, in any of the embodiments described above, image-capturing of a food product and identification of the kind may be performed before or may be performed after radiation of light and measurement of spectroscopy. Also, in any of the embodiments described above, the mobile apparatus may be a special-purpose apparatus for determining a characteristic of a food product instead of a general-purpose information communication terminal.

### [Example 1]

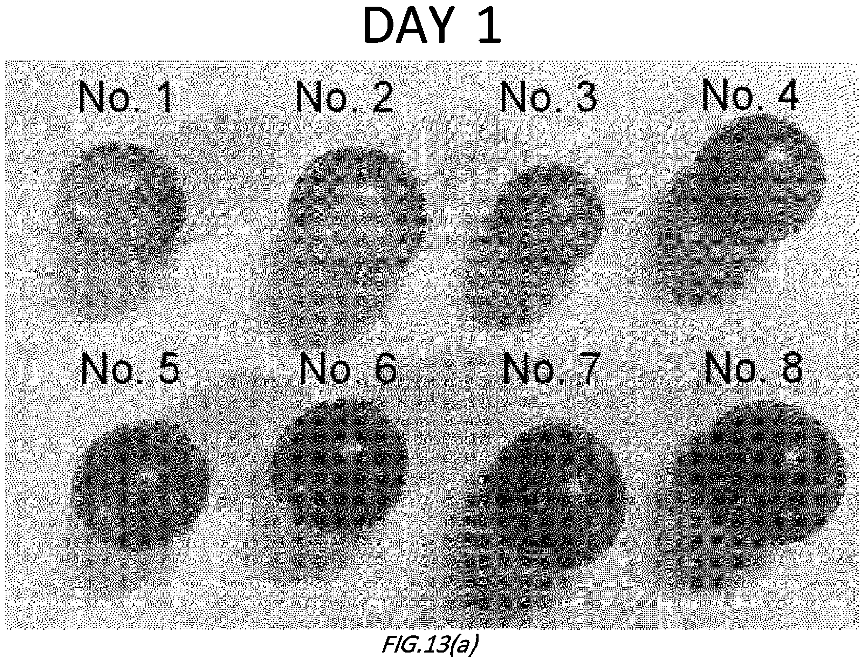

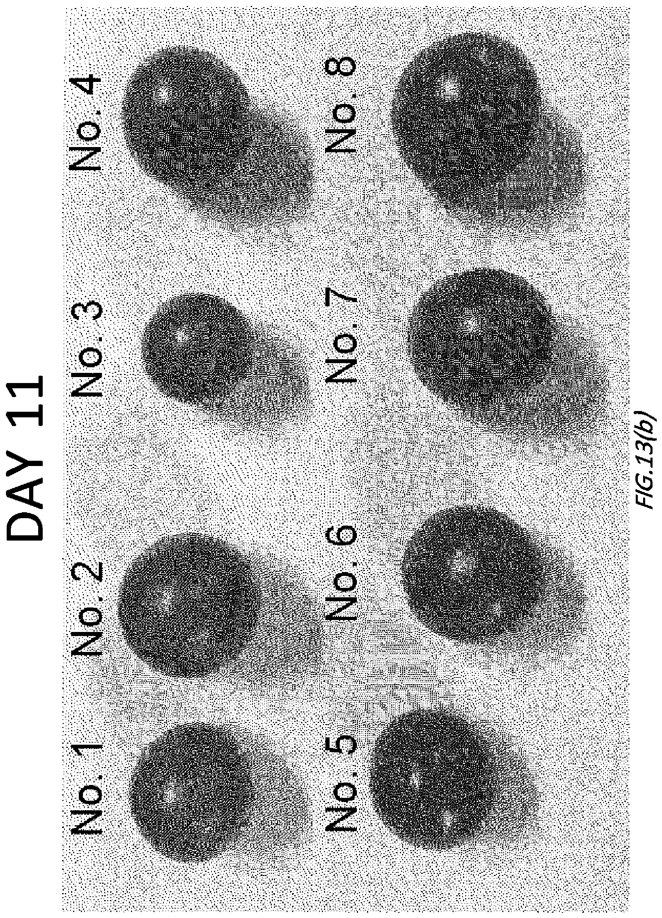

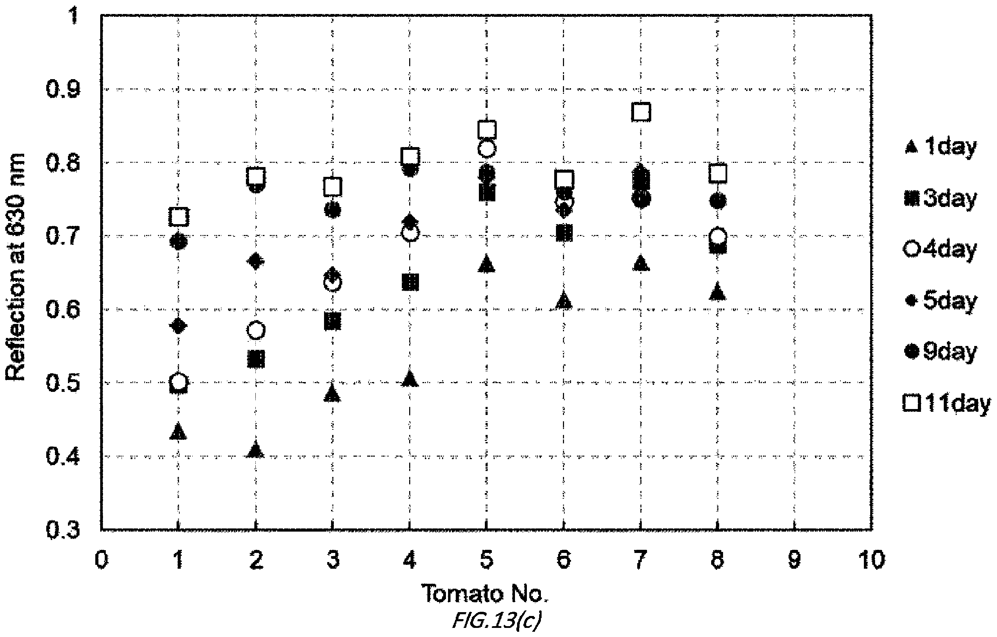

Fig. 13(a), Fig. 13(b), and Fig. 13(c) show an example of determining "Color 1" as a characteristic of "Tomato" as a kind of a food product using the mobile apparatus 10 according to the first embodiment. Fig. 13(a) is a picture on day 1 of the exteriors of tomatoes, each of which is individually identified by numbers 1 to 8, and Fig. 13(b) is a picture on day 11 of the exteriors of the same tomatoes.

Fig. 13(c) shows the measurement result of color 1 of each tomato described above. While the developments of color 1, which is redness, of all of the tomatoes as the days passed are shown in the same figure, it can be read about the result that each of them converges at values like limit values.

### [Example 2]

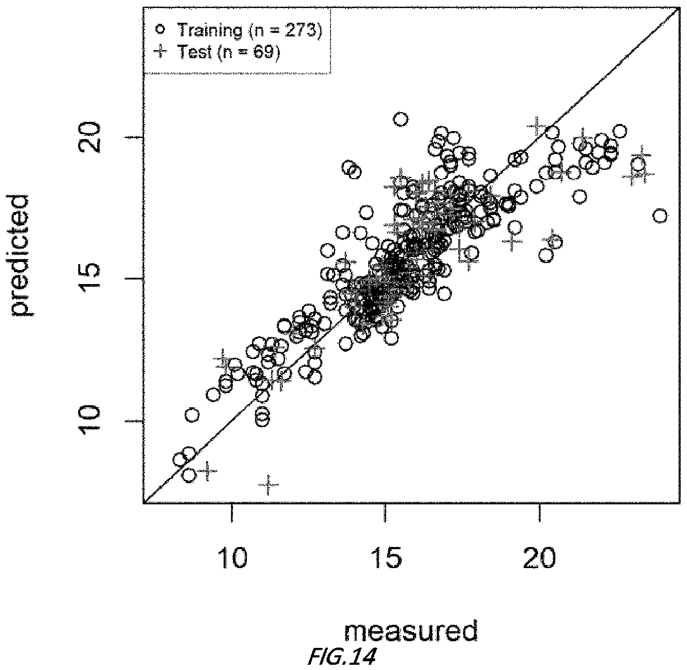

Fig. 14 shows an example of determining "Sugar content" as a characteristic of "Muscat" as a kind of a food product using the mobile apparatus 10 according to the first embodiment. The horizontal axis shows a measured value of sugar content, and the vertical axis shows a prediction value based on a measurement value of the spectroscope 120. Among measured values, "∘" represents a measured value of sugar content by destructive testing. On the other hand, although "+" represents a nondestructive estimated value obtained by performing machine learning using "∘" described above (that is, an actual measured value of destructive testing), it may be considered as a value equivalent to a measured value by destructive testing. Also, as described with reference to Fig. 4, measurement values of the spectroscope 120 and prediction values of sugar content are related in advance.

It can be understood that the accuracy increases as the shape formed by the points on the graph is similar to a shape of a 45-degree straight line. Turning to Fig. 14, points generally gather around the 45-degree straight line.

### [Example 3]

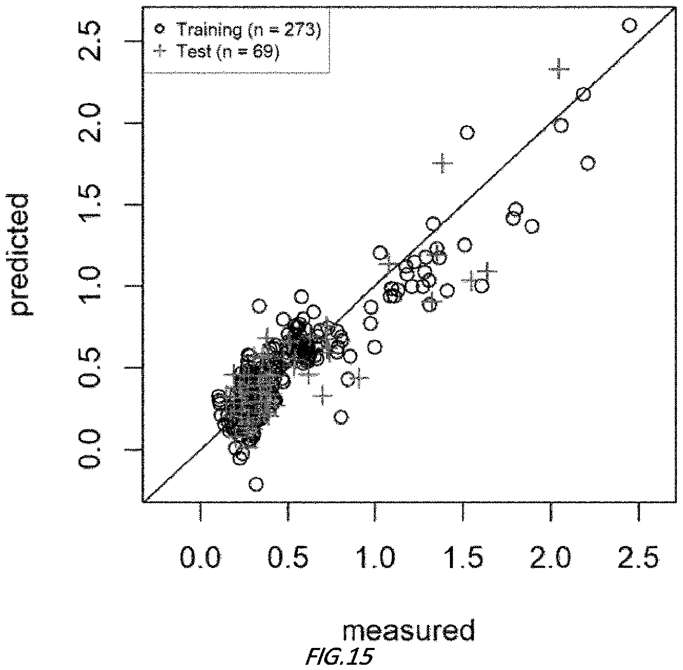

Fig. 15 shows an example of determining "Acidity" as a characteristic of "Muscat" as a kind of a food product using the mobile apparatus 10 according to the first embodiment. The horizontal axis, the vertical axis, "∘", and "+" correspond to those in Fig. 14, but sugar content is replaced with acidity. Although measurement values of the spectroscope 120 and prediction values of acidity are related with each other in advance, the relationship is different from the relationship for sugar content.

It can be understood that the accuracy increases as the shape formed by the points on the graph is similar to a shape of a 45-degree straight line. Turning to Fig. 15, points generally gather around the 45-degree straight line. In addition, as seen from Fig. 13 to Fig. 15, determining characteristics of different kinds of food products, determining different characteristics of the same kind, or the like can easily and precisely performed by using the same mobile apparatus 10.

While the present invention has been described with the embodiments, the technical scope of the present invention is not limited to the above-described embodiments. It is apparent to persons skilled in the art that various alterations and improvements can be added to the above-described embodiments. It is also apparent from the scope of the claims that the embodiments added with such alterations or improvements can be included in the technical scope of the present invention.

Note that the operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the operation flow is described by using phrases such as "first" or "next" in the scope of the claims, specification, or drawings, it does not necessarily mean that the process must be performed in this order.

### EXPLANATION OF REFERENCES

10, 12, 16, 19, 50, 52, 54, 56: mobile apparatus; 14: spectroscopy unit; 18: server; 20: food characteristics determination device; 22: food characteristics determination system; 30, 32: food product; 100: body; 102: sample holder; 104: touch panel; 110: radiating unit; 120, 122: spectroscope; 124: arm; 126: optical system; 130: image-capturing unit; 132: through hole; 140: identification unit; 150: storage unit; 160: determination unit; 170: communication unit; 180: output unit; 200: storage unit; 210: communication unit; 220: identification unit; and 230: determination unit.

## Claims

1. A mobile apparatus, comprising:
a radiating unit (110) configured to radiate light;
a spectroscope (120, 122) configured to perform spectroscopy of light entered to output a measured value;
an identification unit (140) configured to identify a kind of a food product corresponding to the light entered the spectroscope; and
a determination unit (160) configured to determine a characteristic of the food product to output the characteristic of the food product, by referring to a storage unit (150) configured to store a kind of a food product, a characteristic of the food product, and information about light in association with each other, based on the measured value output from the spectroscope and the kind of the food product identified by the identification unit (140); **characterized in that** the spectroscope (120, 122) includes a first spectroscope (122) measuring a transmitted light transmitted through the food product and a second spectroscope (120) measuring a scattered light scattered by the food product, the first spectroscope (122) and the second spectroscope (120) are located in different places from each other.

2. The mobile apparatus according to claim 1, wherein the mobile apparatus is a general-purpose information communication terminal configured to fulfill a function corresponding to an installed application.

3. The mobile apparatus according to claim 2, wherein the spectroscope (120, 122) is detachable.

4. The mobile apparatus according to claim 2 or 3, wherein the radiating unit (110) is detachable.

5. The mobile apparatus according to any one of claims 1 to 4, further comprising:
an image-capturing unit (130) configured to generate an image by image-capturing, wherein
the identification unit (140) is configured to identify the kind of the food product based on the image.

6. The mobile apparatus according to claim 5, wherein
the information about light is stored in the storage unit (150) in further association with a part of the food product,
the identification unit (140) is configured to identify the part of the food product based on the image, and
the determination unit (160) is configured to determine the characteristic of the food product further based on the part of the food product.

7. The mobile apparatus according to any one of claims 1 to 6, wherein the characteristic of the food product includes at least any one of color, sugar content, acidity, maturity, firmness, or a functional ingredient amount.

8. The mobile apparatus according to any one of claims 1 to 7, wherein the radiating unit (110) is configured to radiate light in a visible light band, and the spectroscope (120, 122) is configured to have sensitivity to the visible light band.

9. The mobile apparatus according to any one of claims 1 to 8, wherein the radiating unit (110) is configured to radiate light in a near-infrared band, and the spectroscope (120, 122)is configured to have sensitivity to the near-infrared band.

10. The mobile apparatus according to any one of claims 1 to 9, wherein the radiating unit (110) is configured to radiate light in an ultraviolet band, and the spectroscope (120, 122) is configured to have sensitivity to the ultraviolet band.

11. The mobile apparatus according to any one of claims 1 to 10, wherein
the information about light is stored in the storage unit (150) in further association with a distinction between scattered light and transmitted light, and
the determination unit (160) is configured to determine the characteristic of the food product further based on whether the measured value is of scattered light or transmitted light.

12. A food characteristics determination device, comprising:
a storage unit (150) configured to store a kind of food product, a characteristic of the food product, and information about light in association with each other;
a communication unit (170) configured to communicate with a mobile apparatus; and
a determination unit (160) configured to determine a characteristic of a food product by acquiring a kind of the food product and a measured value of spectroscopy from the mobile apparatus via the communication unit (170) and referring to the storage unit (150), and output the characteristic of the food product to the mobile apparatus via the communication unit (170);
the mobile apparatus includes a spectroscope (120, 122) configured to perform spectroscopy of light entered to output a measured value; **characterized in that**
the spectroscope (120, 122) includes a first spectroscope (122) measuring a transmitted light transmitted through the food product and a second spectroscope (120) measuring a scattered light scattered by the food product, the first spectroscope (122) and the second spectroscope (120) are located in different places from each other.

13. A food characteristics determination method, comprising:
radiating light;
performing spectroscopy of light entered to acquire a measured value;
identifying a kind of a food product corresponding to the light entered in the performing the spectroscopy; and
outputting a characteristic of the food product by determining the characteristic of the food product by referring to a storage unit (150) configured to store a kind of a food product, a characteristic of the food product, and information about light in association with each other, based on the measured value output in the performing the spectroscopy and the kind of the food product identified in the identifying, to determine the characteristic of the food product;
**characterized in that**, in the performing spectroscopy, a first spectroscope (122) measuring a transmitted light transmitted through the food product and a second spectroscope (120) measuring a scattered light scattered by the food product are used, and the first spectroscope (122) and the second spectroscope (120) are located in different places from each other.

14. A program for causing a mobile apparatus in accordance with claim 1 to perform:
radiating light from a radiating unit (110);
performing spectroscopy of light entered to acquire a measured value from a spectroscope (120, 122);
identifying a kind of a food product corresponding to the light entered in the performing the spectroscopy; and
outputting a characteristic of the food product by determining the characteristic of the food product by referring to a storage unit (150) configured to store a kind of a food product, a characteristic of the food product, and information about light in association with each other, based on the measured value output in the performing the spectroscopy and the kind of the food product identified in the identifying, to determine the characteristic of the food product; **characterized in that**
the spectroscope (120, 122) includes a first spectroscope (122) measuring a transmitted light transmitted through the food product and a second spectroscope (120) measuring a scattered light scattered by the food product, the first spectroscope (122) and the second spectroscope (120) are located in different places from each other.

15. The mobile apparatus according to claim 1, wherein
the first spectroscope (122) is arranged in an opposing position to the radiating unit (110) by an arm (124) extending from the radiating unit (110) side, and performs measurement of specularly transmitted light transmitted through the food product, or
the first spectroscope (122) is arranged sideways relative to the radiating unit (110) by an arm (124) extending from the radiating unit (110) side, and performs measurement of diffusely transmitted light transmitted through the food product.

16. The mobile apparatus according to claim 15, wherein the arm (124) has an arc shape.

## Patentansprüche

1. Mobile Einrichtung, umfassend:
eine Bestrahlungseinheit (110), die konfiguriert ist, um ein Licht abzustrahlen;
ein Spektroskop (120, 122), das konfiguriert ist zum Durchführen einer Spektroskopie eines eingetretenen Lichts, um einen gemessenen Wert auszugeben;
eine Identifizierungseinheit (140), die konfiguriert ist zum Identifizieren einer Art eines Lebensmittelprodukts, das dem Licht entspricht, das in das Spektroskop eingetreten ist; und
eine Ermittlungseinheit (160), die konfiguriert ist zum Ermitteln einer Eigenschaft des Lebensmittelprodukts, um die Eigenschaft des Lebensmittelprodukts auszugeben, indem auf eine Speichereinheit (150) Bezug genommen wird, die konfiguriert ist zum Speichern einer Art eines Lebensmittelprodukts, einer Eigenschaft des Lebensmittelprodukts und von Informationen über das damit in Zusammenhang stehende Licht, basierend auf dem gemessenen Wert, der von dem Spektroskop ausgegeben wird, und der Art des Lebensmittelprodukts, das durch die Identifizierungseinheit (140) identifiziert wird; **dadurch gekennzeichnet, dass** das Spektroskop (120, 122) ein erstes Spektroskop (122), das ein Durchlicht misst, welches das Lebensmittelprodukt durchdringt, und ein zweites Spektroskop (120) beinhaltet, das ein Streulicht misst, das von dem Lebensmittelprodukt gestreut wird, wobei sich das erste Spektroskop (122) und das zweite Spektroskop (120) an voneinander getrennten Stellen befinden.

2. Mobile Einrichtung nach Anspruch 1, wobei die mobile Einrichtung ein Mehrzweck-Informationskommunikationsendgerät ist, das konfiguriert ist, um eine Funktion zu erfüllen, die einer installierten Anwendung entspricht.

3. Mobile Einrichtung nach Anspruch 2, wobei das Spektroskop (120, 122) abnehmbar ist.

4. Mobile Einrichtung nach Anspruch 2 oder 3, wobei die Bestrahlungseinheit (110) abnehmbar ist.

5. Mobile Einrichtung nach einem der Ansprüche 1 bis 4, die ferner umfasst:
eine Bilderfassungseinheit (130), die konfiguriert ist zum Erzeugen eines Bildes durch eine Bilderfassung, wobei die Identifizierungseinheit (140) konfiguriert ist, um, basierend auf dem Bild, die Art des Lebensmittelprodukts zu identifizieren.

6. Mobile Einrichtung nach Anspruch 5, wobei:
die Informationen über das Licht in einem weiteren Zusammenhang mit einem Teil des Lebensmittelprodukts in der Speichereinheit (150) gespeichert werden,
die Identifizierungseinheit (140) konfiguriert ist, um den Teil des Lebensmittelprodukts basierend auf dem Bild zu identifizieren, und
die Ermittlungseinheit (160) konfiguriert ist, um die Eigenschaft des Lebensmittelprodukts, ferner basierend auf dem Teil des Lebensmittelprodukts, zu ermitteln.

7. Mobile Einrichtung nach einem der Ansprüche 1 bis 6, wobei die Eigenschaft des Lebensmittelprodukts mindestens eines von einer Farbe, einem Zuckergehalt, einem Säuregehalt, einer Reife, einer Festigkeit oder einer Menge von funktionellen Bestandteilen beinhaltet.

8. Mobile Einrichtung nach einem der Ansprüche 1 bis 7, wobei die Bestrahlungseinheit (110) konfiguriert ist, um Licht in einem sichtbaren Lichtband abzustrahlen, und das Spektroskop (120, 122) konfiguriert ist, um eine Empfindlichkeit gegenüber dem sichtbaren Lichtband aufzuweisen.

9. Mobile Einrichtung nach einem der Ansprüche 1 bis 8, wobei die Bestrahlungseinheit (110) konfiguriert ist, um Licht in einem Nahinfrarotband abzustrahlen, und das Spektroskop (120, 122) konfiguriert ist, um eine Empfindlichkeit gegenüber dem Nahinfrarotband aufzuweisen.

10. Mobile Einrichtung nach einem der Ansprüche 1 bis 9, wobei die Bestrahlungseinheit (110) konfiguriert ist, um Licht in einem Ultraviolettband abzustrahlen, und das Spektroskop (120, 122) konfiguriert ist, um eine Empfindlichkeit gegenüber dem Ultraviolettband aufzuweisen.

11. Mobile Einrichtung nach einem der Ansprüche 1 bis 10, wobei
die Informationen über das Licht in der Speichereinheit (150) in einem weiteren Zusammenhang mit einer Unterscheidung zwischen Streulicht und Durchlicht gespeichert werden, und
die Ermittlungseinheit (160) konfiguriert ist, um die Eigenschaft des Lebensmittelprodukts ferner basierend darauf zu ermitteln, ob der gemessene Wert von dem Streulicht oder dem Durchlicht stammt.

12. Vorrichtung zum Ermitteln von Lebensmitteleigenschaften, umfassend:
eine Speichereinheit (150), die konfiguriert ist, um eine Art eines Lebensmittelprodukts, eine Eigenschaft des Lebensmittelprodukts und von Informationen über das damit in Zusammenhang stehende Licht zu speichern;
eine Kommunikationseinheit (170), die konfiguriert ist, um mit einer mobilen Einrichtung zu kommunizieren; und
eine Ermittlungseinheit (160), die konfiguriert ist, um eine Eigenschaft eines Lebensmittelprodukts zu ermitteln, indem eine Art des Lebensmittelprodukts und ein gemessener Wert einer Spektroskopie von der mobilen Einrichtung über die Kommunikationseinheit (170) und unter Bezugnahme auf die Speichereinheit (150) erfasst werden, und um die Eigenschaft des Lebensmittelprodukts über die Kommunikationseinheit (170) an die mobile Einrichtung auszugeben;
wobei die mobile Einrichtung ein Spektroskop (120, 122) beinhaltet, das konfiguriert ist zum Durchführen einer Spektroskopie eines eingetretenen Lichts, um einen gemessenen Wert auszugeben; **dadurch gekennzeichnet, dass** das Spektroskop (120, 122) ein erstes Spektroskop (122), das ein Durchlicht misst, welches das Lebensmittelprodukt durchdringt, und ein zweites Spektroskop (120) beinhaltet, das ein Streulicht misst, das von dem Lebensmittelprodukt gestreut wird, wobei sich das erste Spektroskop (122) und das zweite Spektroskop (120) an voneinander getrennten Stellen befinden.

13. Verfahren zum Ermitteln von Lebensmitteleigenschaften, umfassend:
Abstrahlen eines Lichts;
Durchführen einer Spektroskopie von eingetretenem Licht, um einen gemessenen Wert zu erfassen;
Identifizieren einer Art eines Lebensmittelprodukts, das dem Licht entspricht, das bei dem Durchführen der Spektroskopie eingetreten ist; und
Ausgeben einer Eigenschaft des Lebensmittelprodukts, indem die Eigenschaft des Lebensmittelprodukts durch eine Bezugnahme auf eine Speichereinheit (150) ermittelt wird, die konfiguriert ist zum Speichern einer Art eines Lebensmittelprodukts, einer Eigenschaft des Lebensmittelprodukts und von Informationen über das damit in Zusammenhang stehende Licht, basierend auf dem gemessenen Wert, der bei dem Durchführen der Spektroskopie ausgegeben wird, und der Art des Lebensmittelprodukts, das bei dem Identifizieren identifiziert wird, um die Eigenschaft des Lebensmittelprodukts zu ermitteln.
**dadurch gekennzeichnet, dass** bei dem Durchführen einer Spektroskopie ein erstes Spektroskop (122), das ein Durchlicht misst, welches das Lebensmittelprodukt durchdringt, und ein zweites Spektroskop (120), das ein Streulicht misst, das von dem Lebensmittelprodukt gestreut wird, verwendet werden, und wobei sich das erste Spektroskop (122) und das zweite Spektroskop (120) an voneinander getrennten Stellen befinden.

14. Programm zum Veranlassen, das eine mobile Einrichtung nach Anspruch 1 geeignet ist zum Durchführen von:
Abstrahlen eines Lichts von einer Bestrahlungseinheit (110);
Durchführen einer Spektroskopie von eingetretenem Licht, um einen gemessenen Wert von einem Spektroskop zu erfassen (120, 122);
Identifizieren einer Art eines Lebensmittelprodukts, das dem Licht entspricht, das bei dem Durchführen der Spektroskopie eingetreten ist; und
Ausgeben einer Eigenschaft des Lebensmittelprodukts, indem die Eigenschaft des Lebensmittelprodukts durch eine Bezugnahme auf eine Speichereinheit (150) ermittelt wird, die konfiguriert ist zum Speichern einer Art eines Lebensmittelprodukts, einer Eigenschaft des Lebensmittelprodukts und von Informationen über das damit in Zusammenhang stehende Licht, basierend auf dem gemessenen Wert, der bei dem Durchführen der Spektroskopie ausgegeben wird, und der Art des Lebensmittelprodukts, das bei dem Identifizieren identifiziert wird, um die Eigenschaft des Lebensmittelprodukts zu ermitteln; **dadurch gekennzeichnet, dass**
das Spektroskop (120, 122) ein erstes Spektroskop (122), das ein Durchlicht misst, welches das Lebensmittelprodukt durchdringt, und ein zweites Spektroskop (120) beinhaltet, das ein Streulicht misst, das von dem Lebensmittelprodukt gestreut wird, wobei sich das erste Spektroskop (122) und das zweite Spektroskop (120) an voneinander getrennten Stellen befinden.

15. Mobile Einrichtung nach Anspruch 1, wobei:
das erste Spektroskop (122) an einer der Bestrahlungseinheit (110) gegenüberliegenden Position durch einen Arm (124) angeordnet ist, der sich von der Seite der Bestrahlungseinheit (110) aus erstreckt, und eine Messung eines gespiegelten Durchlichts durchführt, welches das Lebensmittelprodukt durchdringt, oder
das erste Spektroskop (122) seitlich relativ zur Bestrahlungseinheit (110) durch einen Arm (124) angeordnet ist, der sich von der Seite der Bestrahlungseinheit (110) aus erstreckt, und eine Messung eines diffusen Durchlichts durchführt, welches das Lebensmittelprodukt durchdringt.

16. Mobile Einrichtung nach Anspruch 15, wobei der Arm (124) eine Bogenform aufweist.

## Revendications

1. Appareil mobile, comprenant :
une unité rayonnante (110) conçue pour rayonner de la lumière ;
un spectroscope (120, 122) conçu pour réaliser une spectroscopie sur la lumière entrée afin de délivrer une valeur mesurée ;
une unité d'identification (140) conçue pour identifier un type d'un produit alimentaire correspondant à la lumière entrée dans le spectroscope ; et
une unité de détermination (160) conçue pour déterminer une caractéristique du produit alimentaire afin de délivrer la caractéristique du produit alimentaire, en se référant à une unité de stockage (150) conçue pour stocker un type d'un produit alimentaire, une caractéristique du produit alimentaire et des informations sur la lumière en association les uns avec les autres, en se basant sur la valeur mesurée délivrée par le spectroscope et le type du produit alimentaire identifié par l'unité d'identification (140) ;
**caractérisé en ce que** le spectroscope (120, 122) comporte un premier spectroscope (122) mesurant une lumière transmise à travers le produit alimentaire et un deuxième spectroscope (120) mesurant une lumière diffusée par le produit alimentaire, le premier spectroscope (122) et le deuxième spectroscope (120) étant situés à des endroits différents l'un de l'autre.

2. Appareil mobile selon la revendication 1, l'appareil mobile étant un terminal de communication d'informations à usage général conçu pour remplir une fonction correspondant à une application installée.

3. Appareil mobile selon la revendication 2, dans lequel le spectroscope (120, 122) est détachable.

4. Appareil mobile selon la revendication 2 ou 3, dans lequel l'unité rayonnante (110) est détachable.

5. Appareil mobile selon l'une quelconque des revendications 1 à 4, comprenant en outre :
une unité de capture d'images (130) conçue pour générer une image par capture d'image, dans lequel
l'unité d'identification (140) est conçue pour identifier le type du produit alimentaire en se basant sur l'image.

6. Appareil mobile selon la revendication 5, dans lequel
les informations sur la lumière sont stockées dans l'unité de stockage (150) également en association avec une partie du produit alimentaire,
l'unité d'identification (140) est conçue pour identifier la partie du produit alimentaire en se basant sur l'image, et
l'unité de détermination (160) est conçue pour déterminer la caractéristique du produit alimentaire en se basant également sur la partie du produit alimentaire.

7. Appareil mobile selon l'une quelconque des revendications 1 à 6, dans lequel la caractéristique du produit alimentaire en comporte au moins une des suivantes, quelle qu'elle soit : couleur, teneur en sucre, acidité, maturité, fermeté, ou quantité d'ingrédient fonctionnel.

8. Appareil mobile selon l'une quelconque des revendications 1 à 7, dans lequel l'unité rayonnante (110) est conçue pour rayonner de la lumière dans une bande de lumière visible, et le spectroscope (120, 122) est conçu pour présenter une sensibilité à la bande de lumière visible.

9. Appareil mobile selon l'une quelconque des revendications 1 à 8, dans lequel l'unité rayonnante (110) est conçue pour rayonner de la lumière dans une bande proche infrarouge, et le spectroscope (120, 122) est conçu pour présenter une sensibilité à la bande proche infrarouge.

10. Appareil mobile selon l'une quelconque des revendications 1 à 9, dans lequel l'unité rayonnante (110) est conçue pour rayonner de la lumière dans une bande ultraviolette, et le spectroscope (120, 122) est conçu pour présenter une sensibilité à la bande ultraviolette.

11. Appareil mobile selon l'une quelconque des revendications 1 à 10, dans lequel
les informations sur la lumière sont stockées dans l'unité de stockage (150) également en association avec une distinction entre lumière diffusée et lumière transmise, et
l'unité de détermination (160) est conçue pour déterminer la caractéristique du produit alimentaire en se basant également sur le fait que la valeur mesurée est celle d'une lumière diffusée ou d'une lumière transmise.

12. Dispositif de détermination de caractéristiques d'aliments, comprenant :
une unité de stockage (150) conçue pour stocker un type de produit alimentaire, une caractéristique du produit alimentaire et des informations sur la lumière en association les uns avec les autres ;
une unité de communication (170) conçue pour communiquer avec un appareil mobile ; et
une unité de détermination (160) conçue pour déterminer une caractéristique d'un produit alimentaire en acquérant un type du produit alimentaire et une valeur mesurée de spectroscopie provenant de l'appareil mobile par le biais de l'unité de communication (170) et en se référant à l'unité de stockage (150), et délivrer la caractéristique du produit alimentaire à l'appareil mobile par le biais de l'unité de communication (170) ;
l'appareil mobile comportant un spectroscope (120, 122) conçu pour réaliser une spectroscopie sur la lumière entrée afin de délivrer une valeur mesurée ; **caractérisé en ce que**
le spectroscope (120, 122) comporte un premier spectroscope (122) mesurant une lumière transmise à travers le produit alimentaire et un deuxième spectroscope (120) mesurant une lumière diffusée par le produit alimentaire, le premier spectroscope (122) et le deuxième spectroscope (120) étant situés à des endroits différents l'un de l'autre.

13. Procédé de détermination de caractéristiques d'aliments, comprenant :
le rayonnement de lumière ;
la réalisation d'une spectroscopie sur la lumière entrée afin d'acquérir une valeur mesurée ;
l'identification d'un type d'un produit alimentaire correspondant à la lumière entrée lors de la réalisation de la spectroscopie ; et
la délivrance d'une caractéristique du produit alimentaire par détermination de la caractéristique du produit alimentaire par référence à une unité de stockage (150) conçue pour stocker un type d'un produit alimentaire, une caractéristique du produit alimentaire et des informations sur la lumière en association les uns avec les autres, sur la base de la valeur mesurée délivrée lors de la réalisation de la spectroscopie et du type du produit alimentaire identifié lors de l'identification, afin de déterminer la caractéristique du produit alimentaire ;
**caractérisé en ce que**, lors de la réalisation de la spectroscopie, un premier spectroscope (122) mesurant une lumière transmise à travers le produit alimentaire et un deuxième spectroscope (120) mesurant une lumière diffusée par le produit alimentaire sont utilisés, et le premier spectroscope (122) et le deuxième spectroscope (120) se situent à des endroits différents l'un de l'autre.

14. Programme destiné à amener un appareil mobile selon la revendication 1 à réaliser :
le rayonnement de lumière à partir d'une unité rayonnante (110) ;
la réalisation d'une spectroscopie sur la lumière entrée pour acquérir une valeur mesurée à partir d'un spectroscope (120, 122) ;
l'identification d'un type d'un produit alimentaire correspondant à la lumière entrée lors de la réalisation de la spectroscopie ; et
la délivrance d'une caractéristique du produit alimentaire par détermination de la caractéristique du produit alimentaire par référence à une unité de stockage (150) conçue pour stocker un type d'un produit alimentaire, une caractéristique du produit alimentaire et des informations sur la lumière en association les uns avec les autres, sur la base de la valeur mesurée délivrée lors de la réalisation de la spectroscopie et du type du produit alimentaire identifié lors de l'identification, afin de déterminer la caractéristique du produit alimentaire ; **caractérisé en ce que**
le spectroscope (120, 122) comporte un premier spectroscope (122) mesurant une lumière transmise à travers le produit alimentaire et un deuxième spectroscope (120) mesurant une lumière diffusée par le produit alimentaire, le premier spectroscope (122) et le deuxième spectroscope (120) étant situés à des endroits différents l'un de l'autre.

15. Appareil mobile selon la revendication 1, dans lequel
le premier spectroscope (122) est disposé à une position opposée à l'unité rayonnante (110) par un bras (124) s'étendant depuis le côté de l'unité rayonnante (110), et réalise une mesure de lumière transmise spéculairement à travers le produit alimentaire, ou
le premier spectroscope (122) est disposé latéralement par rapport à l'unité rayonnante (110) par un bras (124) s'étendant depuis le côté de l'unité rayonnante (110), et réalise une mesure de lumière transmise de façon diffuse à travers le produit alimentaire.

16. Appareil mobile selon la revendication 15, dans lequel le bras (124) prend la forme d'un arc.
